# FASCICULE DE BREVET EUROPEEN

(11) **EP 2 167 051 B1**
(45) Date de publication et mention de la délivrance du brevet: **19.01.2011**
(21) Numéro de dépôt: 08760927.7
(22) Date de dépôt: 12.06.2008
(51) Int. Cl.: A61K 9/50, A61K 35/52, A61K 9/52, A61P 15/08, B01J 13/20

(54) **MICROCAPSULES CONTENANT DES SPERMATOZOÏDES DE MAMMIFÈRES, DOSE D'INSÉMINATION LES CONTENANT ET UN PROCÉDÉ POUR LEUR OBTENTION**
MIKROKAPSELN MIT SÄUGETIER-SPERMATOZOIDEN, DIESE ENTHALTENDE BESAMUNGSDOSIS UND VERFAHREN ZU IHRER GEWINNUNG
MICROCAPSULES CONTAINING MAMMALS' SPERMATOZOIDS, INSEMINATION DOSE CONTAINING SAME AND METHOD FOR OBTAINING SAME

(30) Priorité: 14.06.2007 FR 0704244
(43) Date de publication de la demande: 31.03.2010
(73) Titulaire: Cooperative Bretonne D'insemination Artificielle Porcine, 35590 Saint Gilles (FR)
(72) Inventeur: NIHOUARN, Bruno, F-35137 Bedee (FR)
(74) Mandataire: Branger, Jean-Yves
(86) Numéro de dépôt international: PCT/EP2008/057386
(87) Numéro de publication internationale: WO 2008/155285

(56) Documents cités:
- EP-A- 1 693 445
- WO-A-00/61119
- WO-A-2006/106400
- US-A- 4 840 891
- US-B1- 6 596 310
- NEBEL RAYMOND L ET AL: "Spermatozoal microencapsulation for use in artificial insemination of farm animals" APPLICATIONS OF CELL IMMOBILISATION BIOTECHNOLOGY SPRINGER, PO BOX 17, 3300 AA DORDRECHT, NETHERLANDS SERIES : FOCUS ON BIOTECHNOLOGY, 2005, pages 539-548, XP009073250 ISSN: 1-4020-3229-3(H)
- TORRE M L ET AL: "Controlled release of swine semen encapsulated in calcium alginate beads" BIOMATERIALS, ELSEVIER SCIENCE PUBLISHERS BV., BARKING, GB, vol. 21, no. 14, juillet 2000 (2000-07), pages 1493-1498, XP004199070 ISSN: 0142-9612
- TIMBERT R ET AL: "Effect of sole and combined pre-treatments on reserve accumulation, survival and germination of encapsulated and dehydrated carrot somatic embryos" PLANT SCIENCE (SHANNON), vol. 120, no. 2, 1996, pages 223-231, XP002466575 ISSN: 0168-9452
- DINARVAND R ET AL: "Effect of surfactant HLB and different formulation variables on the properties of poly-D,L-lactide microspheres of naltrexone prepared by double emulsion technique" JOURNAL OF MICROENCAPSULATION, vol. 22, no. 2, mars 2005 (2005-03), pages 139-151, XP009094440 ISSN: 0265-2048
- KHANDARE J N ET AL: "Preparation and evaluation of nimesulide niosomes for topical application" INDIAN DRUGS 2001 INDIA, vol. 38, no. 4, 2001, pages 197-202, XP009094333 ISSN: 0019-462X
- ROCA J ET AL: "Challenges in pig artificial insemination" REPRODUCTION IN DOMESTIC ANIMALS, vol. 41, no. Suppl. 2, octobre 2006 (2006-10), pages 43-53, XP002466576 ISSN: 0936-6768

## Description

La présente invention est relative à des microcapsules contenant des spermatozoïdes de mammifères.

Elle concerne également une dose d'insémination les contenant, ainsi qu'un procédé pour l'obtention de ces microcapsules.

En ce qui concerne le cas particulier de la truie, le début des chaleurs a lieu en moyenne 30 heures avant l'ovulation et se traduit physiologiquement par une dilation du col de l'utérus.

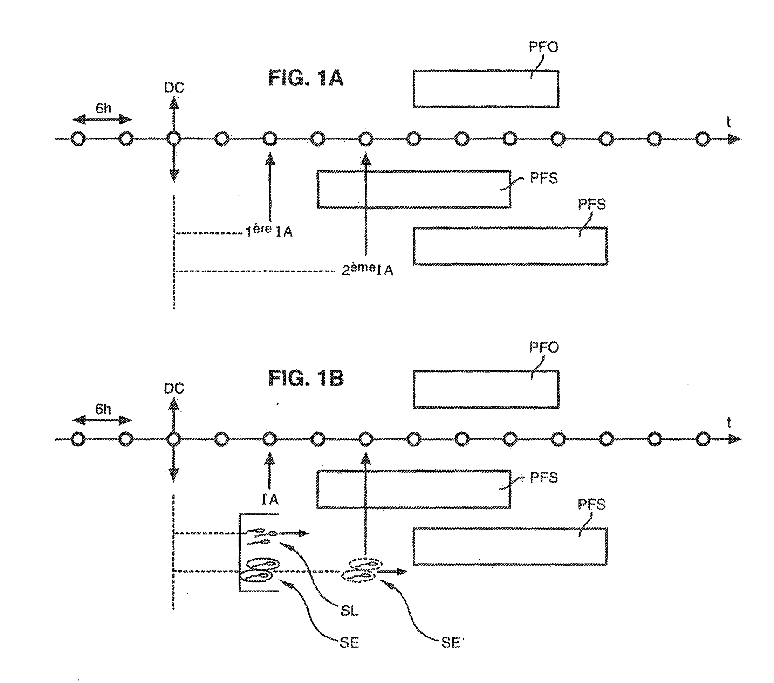

On se reportera à la figure 1A annexée sur laquelle est tracé l'axe du temps t, un intervalle situé en deux cercles voisins représentant 6 heures.

Sur cet axe, le début des chaleurs a été référencé DC et le bloc PFO représente la période de temps pendant laquelle les ovules sont potentiellement fécondables.

Actuellement, la réalisation de l'insémination artificielle est effectuée en deux temps, afin de couvrir la période ovulatoire et la période postovulatoire, ceci afin d'avoir les meilleurs chances de fécondation des ovules.

Une première insémination artificielle (1^{ère} IA) est pratiquée 12 heures après le début des chaleurs DC, ce qui assure une durée fécondante de 24 heures (premier bloc PFS). Une deuxième insémination artificielle (2^{ème} IA) est pratiquée 24 heures après le début des chaleurs, ce qui assure également 24 heures de fécondité (2^{ème} bloc PFS) et qui recouvre alors la période ovulatoire PFO.

On comprend aisément que cette réalisation d'une insémination artificielle en deux temps occasionne de nombreuses manipulations pour le technicien chargé de ces inséminations. Par ailleurs, cela provoque du stress chez la truie. Enfin, ces opérations nécessitent de devoir utiliser plusieurs contenants pour la semence ce qui, globalement, augmente le prix de revient de l'opération.

Il a déjà été proposé de remplacer les deux opérations d'insémination par une seule.

Pour ce faire, on a eu recours à l'encapsulation de spermatozoïdes dans une matrice non toxique qui forme un gel ou un solide à température de stockage, et qui se désagrège à la température corporelle.

Ainsi, on décrit dans le document US-A-4 840 891 l'encapsulation de spermatozoïdes pour l'insémination artificielle.

Dans ce document, on fait usage d'un polymère non toxique hydrophile qui est de préférence choisi dans le groupe constitué des polymères de polyuréthane et de polyoxyéthylène, ainsi que des polymères de polyuréthane et de polyester.

On décrit par ailleurs dans le document EP-B-0 922 451 une technique d'encapsulation de spermatozoïdes de cochon à l'aide de gels de polymères et, particulièrement, d'alginate.

Cette technique ne donne pas de bons résultats dans la mesure où les tests réalisés ne sont pas concluants. En effet, la taille des microcapsules formée est de l'ordre de 5 millimètres, ce qui est beaucoup trop, et la mobilité des spermatozoïdes encapsulés est très inférieure à celle de spermatozoïdes libres.

Dans d'autres documents sont décrites des techniques d'encapsulation de spermatozoïdes. Il s'agit, à titre non limitatif, des documents suivants :
- US-B1-6 596 310,
- US-A-4 840 891,
- WO 2006/106400,
- NEBEL RAYMOND L ET AL : "Spermatozoal microencapsulation for use in artificial insemination of farm animals" APPLICATIONS OF CELL IMMOBILISATION BIOTECHNOLOGY SPRINGER, PO BOX 17, 3300 AA DORDRECHT, NETHERLANDS SERIES : FOCUS ON BIOTECHNOLOGY, 2005, pages 539-548, SP009073250 ISSN : 1-4020-3229-3(H),
- TORRE M L ET AL : "Controlled release of swine semen encapsulated in calcium alginate beads" BIOMATERIALS, ELSEVIER SCIENCE PUBLISHERS BV., BARKING, GB, vol. 21, no. 14, juillet 2000 (2000-07), pages 1493-1498, XP004199070 ISSN : 0142-9612.

Par ailleurs, dans les documents indiqués ci-après sont divulgués des techniques d'encapsulation de matériaux non vivants :
- WO 00/61119 A,
- EP-A-1 693 445,
- TIMBERT R ET AL : "Effect of sole and combined pre-treatments on reserve accumulation, survival and germination of encapsulated and dehydrated carrot somatic embryos" PLANT SCIENCE (SHANNON), vol. 120, no.2, 1996, pages 223-231, XP002466575 ISSN : 0168-9452,
- DINARVAND R ET AL : Effect of surfactant HLB and different formulation variables on the properties of poly-D,L-lactide microspheres of naltrexone prepared by double emulsion technique" JOURNAL OF MICROENCAPSULATION ISSN : 0265-2048,
- KHANDARE J N ET AL : "Preparation and evaluation of nimesulide niosomes for topical application" INDIAN DRUGS 2001 INDIA, vol. 38, no. 4, 2001, pages 197-202, XP009094333 ISSN : 0019-462X.

L'idée d'encapsuler une partie de dose d'insémination nécessite de devoir répondre à certains impératifs. En particulier, l'intégration de la semence dans des capsules doit permettre une libération contrôlée de celle-ci et, par conséquent, le remplacement de la deuxième insémination.

Les capsules produites doivent se maintenir dans le milieu liquide support qui les contient (milieu isotonique), pendant un temps minimum de trois jours, et dans le milieu animal pendant 24 heures. Ces capsules doivent se dégrader en 6 heures dans le milieu animal et doivent avoir une taille d'environ 1 mm.

Dans un but de simplification, on utilisera le terme "dilueur" pour désigner le milieu isotonique dans lequel les capsules sont contenues. Ce dilueur est du type de celui bien connu pour assurer la conservation de semence fraîche pendant plusieurs jours.

En résumé, la matrice d'encapsulation ne doit pas réagir pendant 96 heures au minimum, c'est-à-dire 72 heures dans le milieu isotonique et 24 heures dans le milieu animal.

Cette situation est représentée à la figure 1B annexée, qui est analogue à la précédente. On a référencé IA la seule insémination mise en oeuvre, au cours de laquelle on injecte des spermatozoïdes encapsulés SE.

La référence SE' correspond à la période à partir de laquelle la matrice d'encapsulation commence à se désagréger, la libération complète des spermatozoïdes étant effective au bout de 24h.

Le présent demandeur a constaté que l'utilisation d'une matrice non spermicide constituée d'au moins un glycéride hémi-synthétique, à savoir au moins un glycéride d'acides gras saturés en C₈ à C₁₈, dont la température de fusion est comprise entre environ 35 et environ 47°C, répond tout à fait à l'objectif décrit plus haut.

En effet, une telle matrice d'encapsulation est biocompatible avec la semence, est stable dans le milieu isotonique pendant trois jours, est stable pendant 24 heures dans le milieu animal intra-utérin à des températures de l'ordre de 38°C, et se dégrade en 6 heures environ dans le milieu animal.

La présente invention se rapporte également à une dose d'insémination comprenant un milieu liquide apte à assurer la conservation de spermatozoïdes, cette dose contenant des microcapsules conformes à l'une des caractéristiques précitées.

Selon des formes particulières de réalisation de cette dose :
- ledit milieu liquide contient, en suspension, des spermatozoïdes libres, c'est à dire non encapsulés ;
- elle contient au moins deux groupes de microcapsules, qui se différencient deux à deux par la nature du milieu liquide et/ou celle de ladite matrice ;
- ledit milieu liquide non encapsulé contient un agent apte à permettre la mise en suspension uniforme des microcapsules au sein de celui-ci ;
- ledit agent est un gel ;
- ledit agent est une gomme de gellane.

La présente invention se rapporte également à un procédé de fabrication de telles microcapsules selon lequel on réceptionne les microcapsules dans un bain de solidification.

Selon l'invention, le bain comporte un agent tensioactif.

Selon des caractéristiques particulières de ce procédé :
- ledit agent tensio-actif est un monolaurate de sorbitane ;
- la température du bain de solidification est comprise entre 8 et 25°C.

D'autres caractéristiques et avantages de la présente invention apparaîtront à la lecture détaillée qui va suivre de certains modes de réalisation préférentiels.

Cette description sera faite en référence aux dessins annexés dans lesquels :
- la figure 2 est une vue schématique d'une installation qui permet de fabriquer les microcapsules selon l'invention ;
- la figure 3 est une vue de détail d'une partie de l'installation de la figure 2.

### 1- Sélection de matrices :

Le présent demandeur a testé plusieurs matières grasses et a finalement sélectionné plusieurs matrices aptes à constituer le matériau d'encapsulation des microcapsules selon l'invention.

Il s'agit à chaque fois de matériaux non spermicides, constitués d'au moins un glycéride d'acides gras saturés en C8 à C18, qui présentent une température de fusion comprise entre environ 35° et environ 47°C.

Plus précisément, les produits testés retenus sont ceux les suivants : (désignés sous leurs références commerciales, disponibles auprès de la société GATEFOSSE) : Gelucire 39/01, Gelucire 43/01 et Suppocire DM.

La composition et les caractéristiques de ces produits sont les suivantes :

| | Gelucire 39/01 | Gelucire 43/01 | Suppocire DM |
|---|---|---|---|
| Point de goutte (Mettler) | 37,5 à 41,5 °C | 42,0 à 45,0 °C | 42,0 à 45,0 °C |
| Indice d'acide | < 0,20 mgKOH/g | < 0,20 mgKOH/g | < 0,20 mgKOH/g |
| Indice de saponification | 225 à 245 mgKOH/g | 214 à 236 mgKOH/g | 214 à 236 mgKOH/g |
| Indice d'iode | <2gl2/100g | <2gl2/100g | <2,0gl2/100g |
| Indice d'hydroxyle | < 10 mgKOH/g | < 10 mgKOH/g | < 10 mgKOH/g |
| Indice de péroxyde | < 1,2 meq02/Kg | < 3,0 meq0²/kg | < 3,0 meq0²/kg |
| Impuretés alcalines | < 30 ppm NaOH | < 30 ppm NaOH | < 30 ppm NaOH |
| Teneur en eau | < 0,50 % | < 0,50 % | < 0,50 % |
| Cendres sulfuriques | <0,05% | <0,6% | <0,6% |
| Teneur en | < 0,5 % insaponifiable | < 0,05 % | < 0,05 % |
| Métaux lourds | < 10 ppm | < 10 ppm | < 10 ppm |

Ces matières s'avèrent tout à fait satisfaisantes, en termes de biocompatibilité avec la semence et de température de fusion.

### 2 - Formation de billes à partir des matrices :

L'objectif de ce test est de déterminer les conditions favorables pour la formation de billes lors de l'extrusion des matrices. La solidification des billes doit être rapide et va dépendre du bain de réception où les capsules tombent (Température, tensio-actif, etc.).

Des microcapsules de Gelucire 39/01, extrudées à l'aide d'une seringue, ont été recueillies dans un bain d'eau distillée à une température de 20°, puis de 10°C.

Dans les deux cas, il y a seulement formation d'un film plat à la surface et pas de billes.

Dans ces conditions, plusieurs agents tensio-actifs ont été testés dans le bain de réception de billes, à des différentes concentrations.

Au final, et en particulier pour son rôle non spermicide vis-à-vis de la semence et son absence d'effet néfaste sur la mobilité et la viabilité des spermatozoïdes, le Span 20 (marque déposée) a été sélectionné (il s'agit de monolaurate de sorbitane).

Une concentration de l'ordre de 0,5 à 1 % en poids est particulièrement préférée.

Une synthèse des tests effectués ci-dessus est donnée dans le tableau reporté à la fin de la description.

### 3 - Formation de capsules :

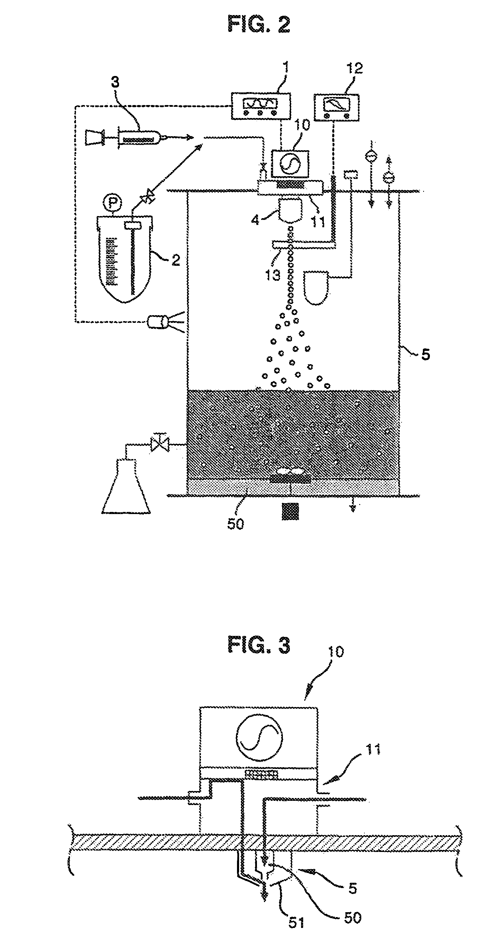

L'équipement utilisé pour les tests d'encapsulation est un appareil appelé "Inotech encapsulator IER -20", qui est représenté schématiquement à la figure 2.

Le principe de cette technique repose sur la désintégration d'un jet laminaire en gouttelettes en lui appliquant une vibration.

L'installation utilisée comporte un générateur de fréquences 1, associé à un dispositif produisant des vibrations 10.

Les matières premières des microcapsules sont respectivement stockées dans un réservoir 2 et une seringue 3 et sont véhiculées jusqu'à une buse 4 raccordée à une chambre à pulsations 11.

Les microcapsules sont recueillies dans un réacteur 5 du type cristallisoir ou bécher, dans le fond duquel est prévu un agitateur magnétique 50 de faible puissance pour maintenir les particules en mouvement.

Un générateur de tension électrique 12 couplé à une électrode 13 permet de charger les microcapsules très fines, les empêchant ainsi de se recoller avant gélification.

La buse 5 est dite à "double buses", à savoir qu'elle comprend une buse "interne" 50 générant le coeur de la microcapsule, et une buse "externe" 51 générant la matrice de la microcapsule (voir figure 3).

### a) Encapsulation du milieu isotonique : dilueur :

Les premiers tests d'encapsulation ont été réalisés avec un dilueur (constitué d'un milieu de conservation de semence animale, enrichi en molécules anti-oxydantes et contenant un antibiotique) et le Gelucire 39/01. Différents paramètres ont dû être ajustés pour obtenir les conditions optimales pour la formation de capsules de taille homogène, en minimisant la perte du dilueur durant la production.

### b) Encapsulation de la semence :

### 1) Préparation de la semence :

L'éjaculât est collecté en fraction riche puis filtrée sur gaze stérile. Un contrôle sur semence pure est effectué sur la concentration (Nucleocounter), la mobilité (SCA) et la morphologie (comptage). La semence est prédiluée (v : v) à 30°C avec du dilueur longue conservation supplémenté en agent de viscosité. La dilution finale est réalisée avec le même dilueur à 20°C. La semence diluée est stabilisée pendant une heure à température ambiante avant d'être stockée à 17°C en attente du process d'encapsulation.

### 2) Optimisation des conditions opératoires :

Plusieurs essais d'encapsulation ont été menés afin d'optimiser les paramètres opératoires suivants :
Le débit de la semence ;
Le débit de la matrice d'encapsulation ;
La fréquence de coupure ;
Le voltage ;
Distance du bain de solidification par rapport aux buses.

Cette optimisation a été basée en partie sur des calculs théoriques qui permettent de prédire les débits pour une fréquence donnée et en fonction des caractéristiques physico-chimiques des matériaux utilisés (viscosité, tension de surface, densité, etc.).

Les capsules formées pour chaque expérience sont visualisées au microscope (taille interne et externe, distribution de taille, forme).

### 4 - Résultats et discussion :

Plusieurs tests d'encapsulation de la semence porcine ont été réalisés sur site pour éviter à la semence de subir les changements de température dus au transport.

### a) Mobilité des spermatozoïdes encapsulés :

La méthode utilisée (tranchage avec un bistouri à lame) pour libérer les spermatozoïdes de la capsule n'est pas très précise et peut provoquer la destruction de la semence lors de la manipulation. Néanmoins, il a été observé dans certains cas que la mobilité des spermatozoïdes encapsulés peut atteindre 65 %.

On estime donc que la semence résiste bien au procédé d'encapsulation et aux variations thermiques provoqués lors de la manipulation.

Dans d'autres cas, une très faible mobilité a été observée, qui peut être due soit à la non-résistance de certaines semences (type de verrats) au procédé d'encapsulation ou à la méthode de bistouri utilisée.

### b) Poids de la capsule :

La masse d'une capsule a été estimée par la pesée d'une centaine de capsules de différents lots. Le poids d'une capsule est compris entre 0,8 et 1,2 mg.
c) Contrôle de libération de spermatozoïdes encapsulés (test in vitro) :

Il a révélé que les spermatozoïdes se libèrent de la capsule dès la première heure de l'incubation. La concentration maximale est atteinte au bout de 20 à 30 heures pour des capsules ayant un diamètre de coeur de 0,6 à 0,9 mm et un diamètre total de 1 mm.

### 5. Mise en suspension des capsules lors de la conservation :

Il a été remarqué que lorsque les billes étaient incubées en présence de la semence, elles ne se répartissaient pas de façon homogène dans la dose et formaient une couche à la surface de la semence diluée. Ceci peut avoir un effet néfaste sur la conservation de la semence.

L'objectif de cette étude est d'ajouter des texturants au milieu (dilueur) pour pouvoir maintenir les billes en suspension.

En raison de ses caractéristiques non spermicides, une gomme de gellane (en anglais "gellan gum") a été sélectionnée. Celle connue sous la marque KELCOGEL LT 100 (sté CP Kerlco) convient parfaitement.
Elle permet un excellent maintien des billes en suspension dès lors qu'elle est présente à une concentration de l'ordre de 0,05 %.

| Tests | Tensio-actif* | Concentration du tensio-actif | Matrice | Formation de capsules | Forme | biocomptabilité | retenu |
|---|---|---|---|---|---|---|---|
| 1 | NI 24-7 | 1% | Gelucire 43/01 | Oui | Sphérique | non | non |
| 2 | NI *45*-*8* | 1% | Gelucire 43/01 | Oui | Sphérique | non | non |
| 3 | Tween 85 | 0,5 % | Gelucire 43/01 | Non | Semi-sphérique | oui | non |
| 4 | Tween 85 | 0,5 % | Gelucire 43/01 | Non | Plane | oui | non |
| *5* | Tween 85 | 1 % | Suppocire 43/01 | Non | Plane | oui | non |
| 6 | Tween *85* | 1 % | Suppocire 43/01 | Non | Plane | oui | non |
| 7 | Tween 20 | 1 % | Gelucire 43/01 | Non | Plane | oui | non |
| 8 | Tween 20 | *0.5*% | Gelucire 43/01 | Non | Plane | oui | non |
| 9 | Span20 | 1% | Gelucire 43/01 | Oui | Sphérique | oui | oui |
| 10 | Span 20 | 1 % | Suppocire DM | Oui | Sphérique | oui | oui |
| 11 | Span20 | 0,5 % | Suppocire DM | Oui | Sphérique | oui | oui |

| | | | | | | | |
|---|---|---|---|---|---|---|---|
| * : Le tensio-actif est identifié par la référence commerciale ou la marque déposée sous laquelle il est disponible | | | | | | | |

## Revendications

1. Microcapsules comprenant :
- un noyau contenant des spermatozoïdes de mammifères, à l'exception de l'être humain, en suspension dans un dilueur, c'est-à-dire un milieu liquide apte à assurer la conservation des spermatozoïdes ;
- une matrice d'encapsulation constituée d'un matériau polymère, **caractérisées par le fait que** ladite matrice est non spermicide et est constituée d'au moins un glycéride hémi-synthétique, à savoir au moins un glycéride d'acides gras saturés en C₈ à C₁₈, qui présente une température de fusion comprise entre environ 35 et environ 47°C.

2. Dose d'insémination comprenant un milieu liquide apte à assurer la conservation de spermatozoïdes, **caractérisée par le fait qu'**elle contient des microcapsules conformes à la revendication 1.

3. Dose selon la revendication 2, **caractérisée par le fait que** ledit milieu liquide contient, en suspension, des spermatozoïdes libres, c'est à dire non encapsulés.

4. Dose selon l'une des revendications 2 ou 3, **caractérisée par le fait qu'**elle contient au moins deux groupes de microcapsules, qui se différencient deux à deux par la nature du milieu liquide et/ou celle de ladite matrice.

5. Dose selon l'un des revendications 2 à 4, **caractérisée par le fait que** ledit milieu liquide non encapsulé contient un agent apte à permettre la mise en suspension uniforme des microcapsules au sein de celui-ci.

6. Dose selon la revendication 5, **caractérisée par le fait que** ledit agent est un gel.

7. Dose selon la revendication 6, **caractérisée par le fait que** ledit agent est une gomme de gellane.

8. Procédé de fabrication de microcapsules selon la revendication 1, selon lequel on réceptionne les dites microcapsules dans un bain de solidification, **caractérisé par le fait que** ledit bain comporte un agent tensio-actif.

9. Procédé selon la revendication 8, **caractérisé par le fait que** ledit agent tensio-actif est un monolaurate de sorbitane.

10. Procédé selon l'une des revendications 9 et 10, **caractérisé par le fait que** la température du bain de solidification est comprise entre 8 et 25°C.

## Claims

1. Microcapsules comprising:
- a core containing spermatozoids of mammals excepting human beings, in suspension in a diluter i.e. a liquid medium capable of ensuring preservation of the spermatozoids;
- an encapsulating matrix comprising a polymer material,
**characterized by** the fact that said matrix is non-spermicidal and consists of at least one semi-synthetic glyceride, namely at least one glyceride of C₈ to C₁₈ saturated fatty acids, which has a melting point of between about 35 and about 47°C.

2. Insemination dose comprising a liquid medium capable of ensuring preservation of spermatozoids, **characterised by** the fact that it contains microcapsules conforming to claim 1.

3. The dose according to claim 2, **characterized by** the fact that said liquid medium contains free spermatozoids in suspension i.e. non-encapsulated.

4. The dose according one of claims 2 or 3, **characterized by** the fact that it contains at least two groups of microcapsules, differing two by two through the type of liquid medium and/or type of said matrix.

5. The dose according to any of claims 2 to 4, **characterized by** the fact that said non-encapsulated liquid medium contains an agent capable of allowing the placing in uniform suspension of the microcapsules therein.

6. The dose according to claim 5, **characterized by** the fact that said agent is a gel.

7. The dose according to claim 6, **characterized by** the fact that said agent is a gellan gum.

8. Method for manufacturing microcapsules according to claim 1, wherein said microcapsules are received in a solidification bath, **characterized by** the fact that said bath comprises a surfactant.

9. The method according to claim 8, **characterized by** the fact that said surfactant is a sorbitan monolaurate.

10. The method according to either of claims 9 and 10, **characterized by** the fact that the temperature of the solidification bath lies between 8 and 25°C.

## Patentansprüche

1. Mikrokapseln, umfassend:
- einen Kern, der Spermatozoide von Säugern mit Ausnahme des Menschen in Suspension in einem Verdünnungsmittel umfasst, das heißt in einem flüssigen Medium, das geeignet ist, die Konservierung der Spermatozoiden sicherzustellen;
- eine Einkapselungsmatrix, die aus einem polymeren Material besteht, **dadurch gekennzeichnet, dass** die Matrix nicht spermizid ist und mindestens aus einem halbsynthetischen Glycerid besteht, nämlich mindestens einem Glycerid von gesättigten C₈- bis C₁₈-Fettsäuren, das einen Schmelzpunkt zwischen etwa 35 und etwa 47 °C einschließlich aufweist.

2. lnseminationsdosis, umfassend ein flüssiges Medium, das geeignet ist, die Konservierung von Spermatozoiden sicherzustellen, **dadurch gekennzeichnet, dass** sie Mikrokapseln enthält, die dem Anspruch 1 entsprechen.

3. Dosis nach Anspruch 2, **dadurch gekennzeichnet, dass** das flüssige Medium in Suspension freie Spermatozoide, das heißt nicht-eingekapselte, enthält.

4. Dosis nach einem der Ansprüche 2 oder 3, **dadurch gekennzeichnet, dass** sie mindestens zwei Gruppen von Mikrokapseln enthält, die sich paarweise durch die Natur des flüssigen Mediums und/oder jener der Matrix unterscheiden.

5. Dosis nach einem der Ansprüche 2 bis 4, **dadurch gekennzeichnet, dass** das nicht-eingekapselte flüssige Medium ein Mittel enthält, das geeignet ist, das gleichmäßige Suspendieren der Mikrokapseln in demselben zu gestatten.

6. Dosis nach Anspruch 5, **dadurch gekennzeichnet, dass** das Mittel ein Gel ist.

7. Dosis nach Anspruch 6, **dadurch gekennzeichnet, dass** das Mittel ein Gellangummi ist.

8. Verfahren zur Herstellung von Mikrokapseln nach Anspruch 1, gemäß welchem man die Mikrokapseln in einem Erstarrungsbad empfängt, **dadurch gekennzeichnet, dass** das Bad ein Tensid umfasst.

9. Verfahren nach Anspruch 8, **dadurch gekennzeichnet, dass** das Tensid ein Sorbitanmonolaurat ist.

10. Verfahren nach einem der Ansprüche 9 und 10, **dadurch gekennzeichnet, dass** die Temperatur des Erstarrungsbades zwischen 8 und 25 °C einschließlich liegt.
